(19) Europäisches Patentamt
European Patent Office
Office européen des brevets

(11) EP 3 884 864 A1

(12) **EUROPEAN PATENT APPLICATION**
published in accordance with Art. 153(4) EPC

(43) Date of publication:
29.09.2021 Bulletin 2021/39

(51) Int Cl.:
*A61B 5/1455* (2006.01)   *G01N 21/17* (2006.01)
*G01N 21/47* (2006.01)

(21) Application number: 19886131.2

(22) Date of filing: 20.11.2019

(86) International application number:
PCT/JP2019/045477

(87) International publication number:
WO 2020/105682 (28.05.2020 Gazette 2020/22)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 21.11.2018 JP 2018218318

(71) Applicant: Medical Photonics Co., Ltd.
Sapporo-shi, Hokkaido 001-0021 (JP)

(72) Inventor: IINAGA, Kazuya
Sapporo-shi, Hokkaido 001-0021 (JP)

(74) Representative: Vossius & Partner
Patentanwälte Rechtsanwälte mbB
Siebertstraße 3
81675 München (DE)

(54) **DEVICE FOR MEASURING BLOOD LIPID CONCENTRATION AND METHOD THEREFOR**

(57) [Problem] To provide a device for measuring the concentration of lipid by a noninvasive lipid measurement and a method therefor. [Solution] The present invention includes: an irradiation unit that irradiates a living body with light with the angle between the surface of the living body and the optical axis being a predetermined angle of 45 to 175°; a light intensity detection unit that is positioned at a predetermined distance from the irradiation unit and measures light intensity reflected from the living body; and a control unit that calculates the amount of turbulence of blood flow on the basis of the light intensity and calculates lipid concentration on the basis of the amount of turbulence of blood flow.

FIG. 1

## Description

Technical Field

The present invention relates to a device for measuring the blood lipid concentration and a method therefor.

Background Art

It is a challenge to suppress the national medical expenses. The cost of treating diseases resulting from lifestyle-related diseases accounts for one-third of the medical expenses. To suppress the national medical expenses, it is required to improve healthy life expectancy and QOL. To this end, the idea of disease prevention has been increasingly popular along with enforcement of designated medical checkups.

It is particularly known that metabolic syndromes, which a designated medical checkup screening is intended to, develop diabetes, dyslipidemias, and hypertension resulting from metabolic disorders caused by accumulation of visceral fat obesity. Early detection of metabolic syndromes is expected to lead to prevention of serious conditions, improvement in QOL, and suppression of the national medical expenses.

Regardless of the fact that metabolic disorders play a critical role in early detection of lifestyle-related diseases as described above, the designated medical checkups only rely on prediction of the risk of insulin resistance based on abdominal circumference size measurement. The reason for this is that it is difficult for the conventional test method using blood collection to instantly measure the metabolism of the living body and it is in addition difficult to measure the in-blood components without blood collection.

Blood lipid, due to its high hydrophobicity, form micelle covered with amphiphilic phospholipid and are present in a particulate form. Blood lipid is called lipoprotein because lipoprotein binds to the surface of the blood lipid.

Lipoprotein is broadly classified into four types in terms of specific gravity. Lipoprotein is classified into chylomicron (CM), VLDL, LDL, and HDL in ascending order of specific gravity. Lipoprotein is also classified into CM, VLDL, LDL, and HDL in descending order of particle size.

Lipoprotein is a collection of cholesterol and triglyceride (TG). Blood tests measure triglyceride and cholesterol, which are the smallest units of the components that form each lipoprotein molecule.

For example, LDL cholesterol called bad cholesterol is the concentration of the cholesterol contained in LDL particles, and measured TG in LDL particles provides LDL-TG. Among a variety of types of cholesterol, LDL cholesterol and HDL cholesterol are known to be indicators relating to arteriosclerosis.

In recent years, postprandial hyperlipidemia has been an important symptom in metabolic measurement. Attention has been directed to postprandial hyperlipidemia as a risk factor of the arteriosclerosis. There has been a report stating that an increase in the concentration of triglyceride in a non-hunger state increases the risk of development of an event of coronary artery diseases.

To diagnose the postprandial hyperlipidemia, however, it is necessary to observe a change in blood lipid concentration for 6 to 8 hours after meals. That is, to measure the state of postprandial hyperlipidemia, it is necessary to place a subject under restraint for 6 to 8 hours and collect blood multiple times. The diagnosis of the postprandial hyperlipidemia is therefore no better than clinical studies, and diagnosing the postprandial hyperlipidemia at a clinical site is not practical.

Patent Literature 1 discloses an approach to a solution of the problem described above. Patent Literature 1 allows measurement of blood lipid not only in a medical institution but at home by eliminating blood collection. Allowing instantaneous data acquisition allows temporally continuous blood lipid measurement.

Citation List

Patent Literature

Patent Literature 1: International Publication No. 2014/087825

Summary of Invention

Technical Problem

The method described in Patent Literature 1 provides generally good results of variation in intra-day blood lipid of the same person. Measured values vary in some cases, however, due to individual differences in the measurement performed on a plurality of persons or differences in measurement sites, resulting in a problem in data sharing or reference value setting. Further, the method described in Patent Literature 1, in which the amount of change in scattering coefficient = the amount of change in particle diameter = amount of change in TG concentration (ΔTG), only measures the amount of change in lipid concentration. It is therefore necessary to develop a technology that allows measurement of the absolute value of lipid concentration (TG).

The present invention has been made to solve the problem with the related art and provides a device for measuring the absolute value of lipid concentration based on using noninvasive lipid concentration measurement and a method therefor.

Solution to Problem

A blood lipid concentration measurement device according to the present invention includes a light radiator that radiates light to a living body at a predetermined angle that is an angle between a surface of a living body and an optical axis, the angle being greater than or equal to 45° but smaller than or equal to 175°, an optical intensity detector that is located at a predetermined distance from the light radiator and measures an optical intensity of the light reflected off the living body, and a controller that calculates an amount of turbulence of blood flow based on the light intensity and calculates a lipid concentration based on the amount of turbulence of blood.

A blood lipid concentration measurement method according to the present invention includes a light radiation step of radiating light to a predetermined position on a living body at a predetermined angle that is an angle between a surface of the living body and an optical axis, the angle being greater than or equal to 45° but smaller than or equal to 175°, an optical intensity detection step of measuring an optical intensity of the light reflected off the living body in a position separate from the predetermined position by a predetermined distance, and the step of calculating an amount of turbulence of blood flow based on the light intensity and calculating a lipid concentration based on the amount of turbulence of blood.

Advantageous Effects of Invention

The blood lipid concentration measurement device according to the present invention and a method for operating the blood lipid concentration measurement device allow measurement of lipid concentration.

Brief Description of Drawings

[Figure 1] Figure 1 shows the configuration of a blood lipid concentration measurement device according to an embodiment of the present invention.
[Figure 2] Figure 2 is a block diagram showing the configuration of a control system of the blood lipid concentration measurement device according to the present embodiment.
[Figure 3] Figure 3 shows that blood lipid scatters light.
[Figure 4] Figure 4 is a flowchart of a blood lipid concentration measurement method according to the present embodiment.
[Figure 5] Figure 5 shows skin, blood, and muscle present in the optical path of light from a light radiator to a light receiver through a living body.
[Figure 6] Figure 6 shows measured individual differences among persons.
[Figure 7] Figure 7 shows the result of frequency analysis using fast Fourier transform (FFT) performed on the result of noninvasive lipid continuous monitoring (six-hour measurement) in a lipid load test.
[Figure 8] Figure 8 shows the results of measurement of temporal changes in blood scatter coefficient.
[Figure 9] Figure 9 shows the results of measurement of turbulence intensity, that is, a temporal change in turbulence of blood flow.
[Figure 10] Figure 10 shows the correlation between the turbulence intensity and the amount of change in TG.
[Figure 11] Figure 11 is a temporal change in received light intensity.
[Figure 12] Figure 12 shows the correlation between the received light intensity and lipid concentration.
[Figure 13] Figure 13 shows the correlation between the received light intensity and the lipid concentration.
[Figure 14] Figure 14 shows verification of a change in turbidity of the blood and the angle of incidence of the light incident on the living body.

Description of Embodiment

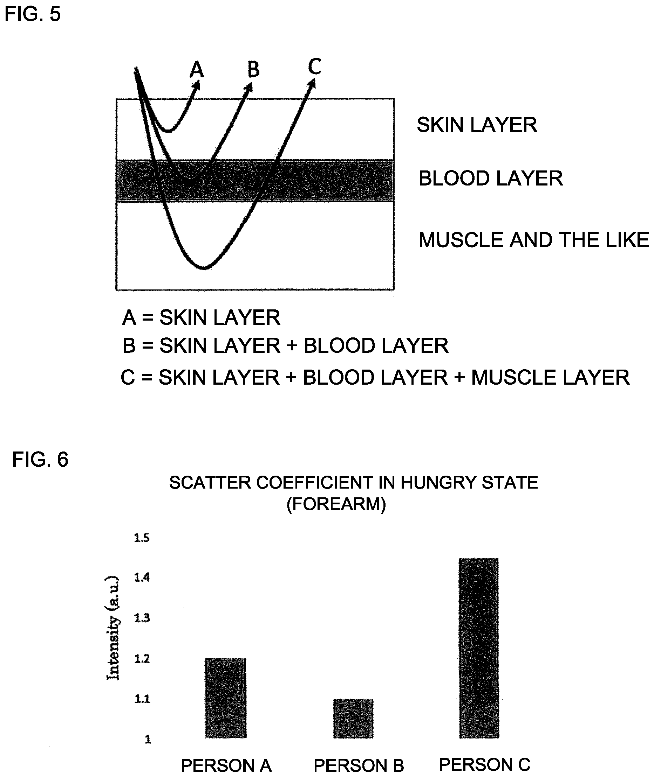

Measurement using light, as shown in Figure 5, provides information on all sites, such as skin, blood, and muscles, present in the path of the light from a light radiator to a light receiver through a living body, and it is therefore necessary to remove information on the skin, muscles, and other sites in order to obtain information only from the blood.

Figure 6 shows measured individual differences among persons. In actual noninvasive measurement, a change in scatter intensity by 0.1 (a.u.) corresponds to a change in TG conversion concentration of about 150 mg/dL. Comparison of measurement results at present in terms of absolute value shows that the results of persons B and C in Figure 6 differ from each other by at least 400 mg/dL, but analyzed results after blood collection show that the actual difference is only about 20 mg/dL.

The present inventor then focused on the fact that the blood keeps flowing in the actual living body and tried to extract only blood information based on the blood motion.

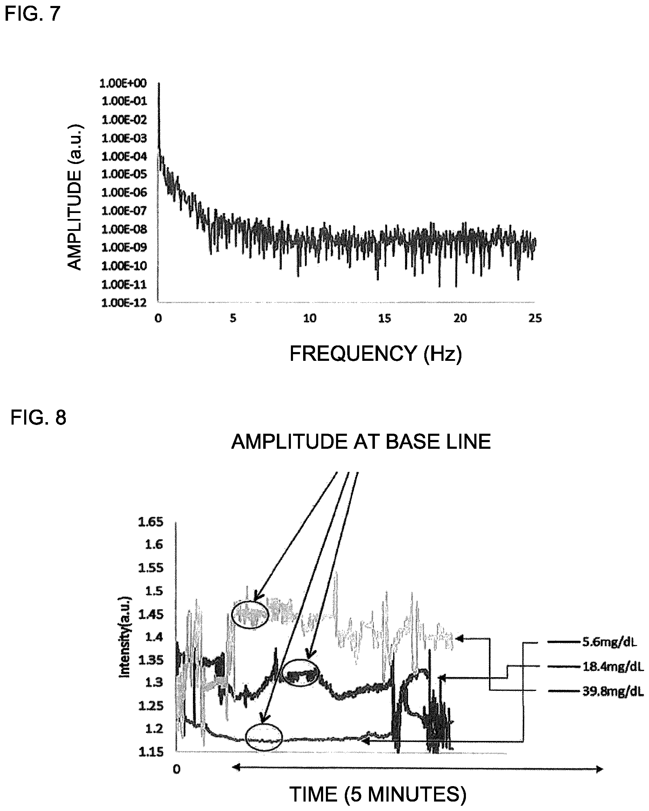

Figure 8 shows the results of measurement of temporal changes in blood scatter coefficient (amount of turbulence of blood flow). Comparison between temporal changes in scatter coefficient (amount of turbulence of blood flow) at different concentrations of chylomicron, which greatly affects optical quantities, shows that the higher the chylomicron concentration, the wider the amplitude of the base lien of the scatter coefficient, as shown in Figure 8.

Figure 7 shows the result of frequency analysis using fast Fourier transform (FFT) performed on the result of noninvasive lipid continuous monitoring (six-hour measurement) in a lipid load test to locate a cause of the amplitude of the base line.

No frequency characteristic was found in the lipid load test, as shown in Figure 7. That is, it can also be said that the amplitude of the base line shown in Figure 8 is random noise.

That is, in the scatter measurement, periodic variation inherent in the living body, such as the heartbeat and respiration as representative examples, is not detected. The reason for this is that measurement at two or more points cancels the periodic variation inherent in the blood.

Further, what is typically called vasomotion or blood flow fluctuation in a long period ranging from 0.1 to 0.2 Hz is observed in blood flow measurement, whereas no vasomotion is observed in the scatter measurement, which also indicates that the periodic change inherent in the living body is canceled.

It is therefore believed that the amplitude seen in Figure 8 is an amplitude resulting from a random change in blood flow. Under this assumption, the present inventor focused on turbulent blood flow.

It is believed that Reynold's number of the blood flowing through the body is about 2000, and the blood flow is typically laminar flow. That is, the blood flow in the body can be taken as stable, organized blood flow. On the other hand, a phenomenon in which the average diameter of the blood lipid particles increases occurs in the state in which the lipid concentration increases after a meal, so that the density of substances including lipid and the like contained in the blood increases. Since Reynold's number increases as the density of substances in a fluid increases, it is believed that the blood flow after a meal has a large Reynold's number and the state of the blood flow changes from the laminar flow to the turbulent flow. It is said that laminar flow changes to turbulent flow when Reynold's number becomes greater than 2300, and it can be said that the blood flow is about to reach a turning point where the state of the blood flow is likely to change from laminar flow to turbulent flow and vice versa.

It is known that when the blood flow in the body changes from laminar flow to turbulent flow, the blood cells and other blood components are deformed as the shear rate increases. It is speculated that the shape of lipoprotein also randomly changes. That is, in optical scatter measurement, a change in the shape of lipoprotein particles due to a turbulent flow can be measured as fluctuation of the measured optical intensity (amount of turbulence of blood flow).

Figure 8 shows the results of the lipid load test and can ascertain that the width between the maximum and the minimum of the amplitude at the base line increases as the amount of chylomicron increases. A considerable reason for this is that an increase in the number of large particles in the blood, such as chylomicron particles, increases the magnitude of the turbulent flow in the blood vessel.

A turbulence intensity I of the blood flow (amount of turbulence of blood flow) in a blood vessel can be determined as follows: Variation σS in the scatter coefficient in a sampling segment τ in noninvasive lipid measurement is determined by the expression below.

[Numerical expression 1]

$$\sigma S = \sqrt{S^2 + \overline{S}^2}$$

S : represents a measured scatter coefficient in the measurement segment τ

S : represents the average of the scatter coefficients in the measurement segment τ

The turbulence intensity I (amount of turbulence of blood flow) is determined as follows:

[Numerical expression 2]

$$I = \frac{\sigma S}{\bar{S}}$$

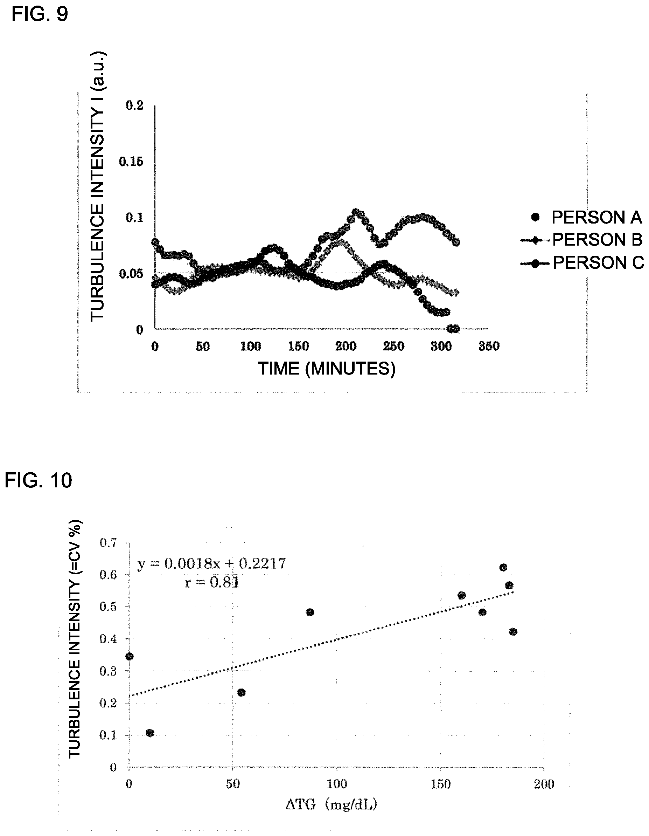

Figure 9 shows the results of measurement of the turbulence intensity, that is, a temporal change in turbulence of the blood flow. Figure 9, which shows measured turbulence of the blood flow, demonstrates that the measurement was made with no individual difference among the persons.

Figure 10 shows the correlation between the turbulence intensity (amount of turbulence of blood flow) and the amount of change in TG, and the resultant correlation coefficient of 0.81 shows that a good correlation is achieved.

As a method for evaluating variation in the optical intensity received for a fixed time, an evaluation indicator, such as a variation coefficient, may be used.

The measurement of the turbulence intensity I can be made with a light receiver provided at a single location, and using light receivers provided at two or more locations readily allows noise cancellation.

Further, the turbulence intensity (amount of turbulence of blood flow) changes the accuracy of information produced at the sampling rate of the device. For example, when a periodic light source, such as a fluorescent lamp, is used as a light source, the accuracy of the information is improved by employing a sampling rate that allows measurement of the period.

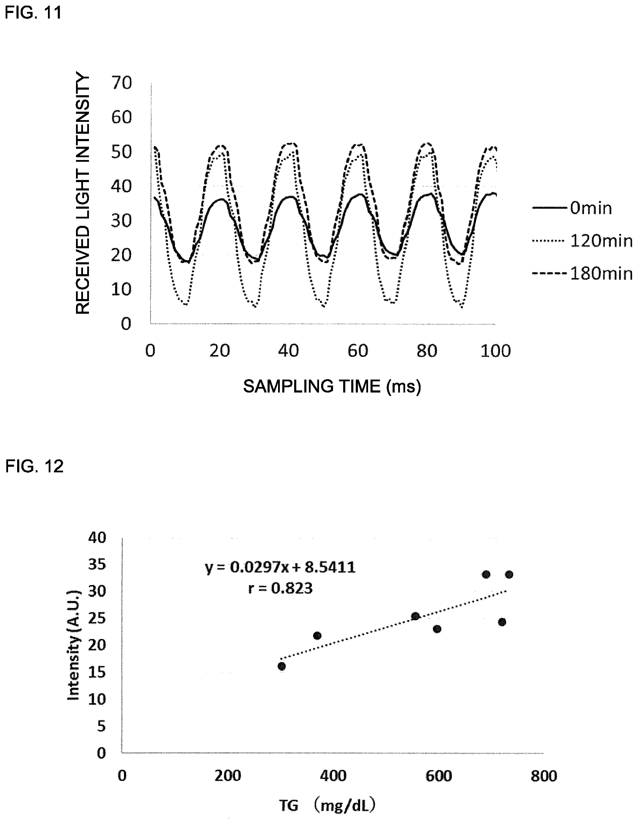

It is, for example said that use of a 50-Hz fluorescent lamp as the light source theoretically allows measurement of the period at a sampling rate of 10 msec. In the present example, the sampling rate is 1 msec, and Figure 11 shows the result of the measurement. Further, in the present embodiment, the measurement time is synonymous with the sampling rate described above. In actual operation, light intensity data to be acquired at the sampling rate described above may be acquired for several minutes to several tens of minutes or otherwise collected, and the resultant light intensity may be averaged or otherwise processed.

Another embodiment may relate to a wearable device that keeps acquiring data continuously and divides the data to acquire optical intensity data from a divided data. Further, a noise portion may be removed from the continuously acquired data or any other data, and the resultant data may be used or otherwise processed.

Figure 11 is a temporal change in received light intensity obtained when a fat load test is performed. The height of the amplitude varies as the lipid concentration increases. Figure 11 also shows that the lower limit of the waveform corresponding to 120 minutes lowers as well as turbulences occur at the top and bottom of the waveform. A considerable reason for this is that unevenness occurs in the optical path as the number of lipid particles increases.

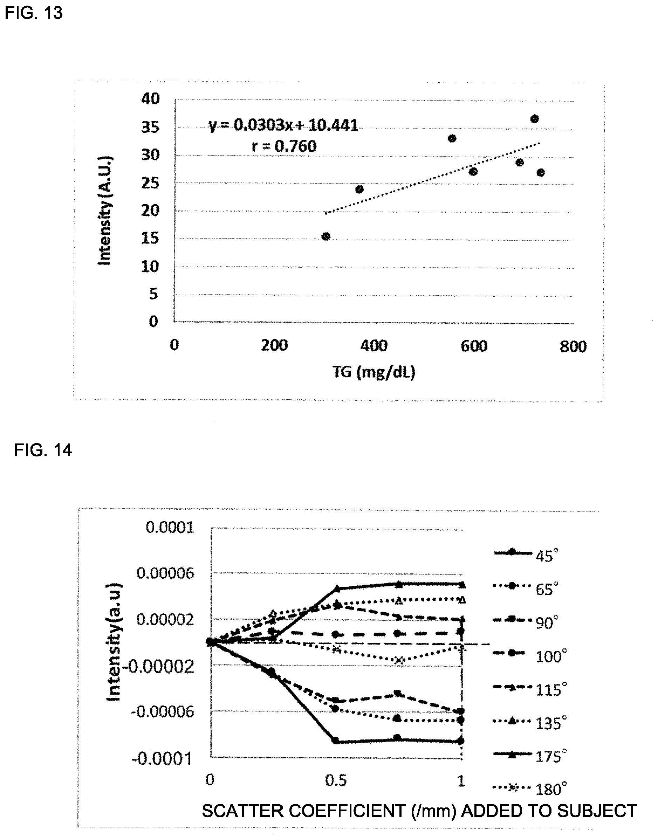

Assuming that the turbulence intensity obtained as described above (amount of turbulence of blood flow) is the width of the amplitude, the correlation shown in Figures 12 and 13 is drawn. Figure 12 shows the correlation for a wrist, and Figure 13 shows the correlation for a forearm. A correlation coefficient of about 0.8, which means a good correlation, is obtained in each of the two cases.

According to the present embodiment, the absolute value of the lipid concentration can be measured by measuring the amount of turbulence of the blood flow. As a result, the number of applications of the device according to the present embodiment increases, and the individual difference in the lipid concentration measurement can be reduced.

A lipid concentration measurement device that is the embodiment of the present invention will next be described in detail with reference to the drawings.

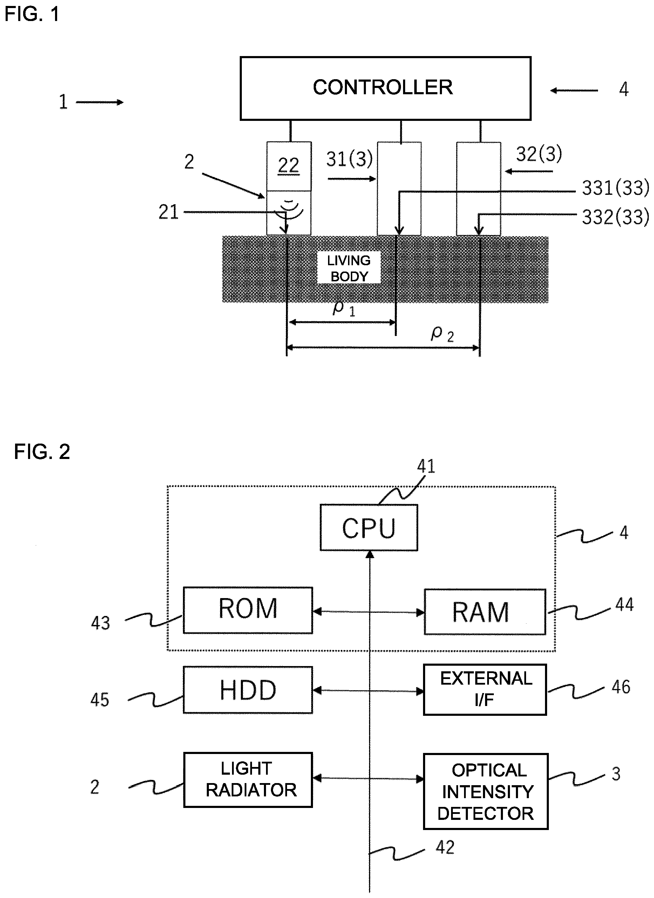

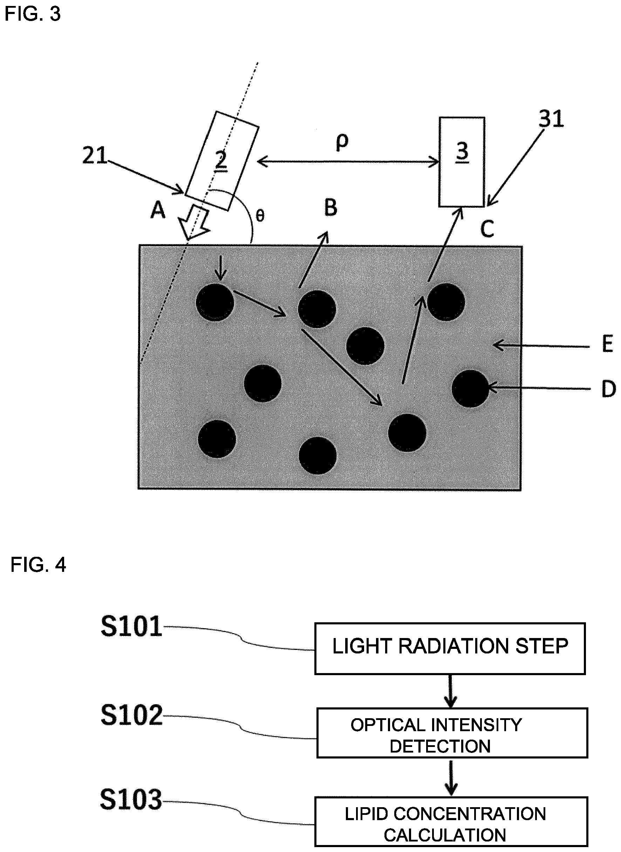

Figure 1 is a block diagram showing the configuration of the blood lipid concentration measurement device according to the present embodiment. A blood lipid concentration measurement device 1 includes a light radiator 2, which radiates radiated light from a point outside a living body toward the living body (A in Figure 3), an optical intensity detector 3 (31, 32), which detects optical intensities at predetermined detection positions 331 and 332 outside the living body, and a controller 4, which calculates the amount of turbulence of the blood flow in the living body based on the optical intensities detected by the optical intensity detector 3 and calculates the lipid concentration based on the amount of turbulence of the blood, as shown in Figure 1.

The light radiator 2 includes a light source 22 for radiating the radiated light to a predetermined radiation position 21 from the point outside the living body toward the interior of the living body, as shown in Figure 1. The light source 22 in the present embodiment can adjust the wavelength of the radiated light. The light source 22 can adjust the range of the wavelength in such a way that the adjusted wavelength range does not fall within the range of the wavelengths at which the light is absorbed by inorganic substances of the blood plasma. The light source 22 can perform the adjustment in such a way that the wavelength range does not fall within the range of the wavelengths at which the light is absorbed by the cell components of the blood. The cell components of the blood used herein are the red blood cells, white blood cells, and platelets in the blood. The inorganic substances of the blood plasma are water and electrolytes in the blood.

The light radiator 2 in the present embodiment can arbitrarily adjust the time length of light radiation, for example, continuous light radiation or pulsed light radiation in accordance with a method for calculating a scatter coefficient $\mu_s'$

calculated by a scatter coefficient calculator 4, which will be described later. The light radiator 2 can arbitrarily modulate the intensity or phase of the radiated light. The intensity of the light radiated from the light radiator 2 is greater than or equal to 0.0025 mW but smaller than or equal to 30 mW. Setting the intensity of the light radiated from the light radiator 2 at a value greater than or equal to 0.0025 mW allows detection of the radiated light emitted from the living body to the space outside the living body. The intensity of the radiated light is preferably smaller than or equal to 30 mW from the viewpoint of safety.

The optical intensity detector 3 receives the radiated light emitted from the living body toward the space outside the living body and detects the optical intensity of the radiated light. In a case where a plurality of optical intensity detectors 3 are used, the optical intensity detectors 3 are so disposed that different positions 331 and 332 roughly around the radiation position 21 are irradiated with the radiated light. In the present embodiment, a first optical intensity detector 31 and a second optical intensity detector 32 are sequentially arranged linearly on the same surface in positions separate from the radiation position 21 and separate from each other at predetermined intervals, as shown in Figure 1. The optical intensity detector 3 may be a photodiode, a CCD, a CMOS device, or any other light receiving element.

In the present embodiment, the distance from the radiation position 21 to the first detection position 331, where the first optical intensity detector 31 performs the detection, is called a first radiation-detection distance $\rho 1$, and the distance from the radiation position 21 to the second detection position 332, where the second optical intensity detector 32 performs the detection, is called a second radiation-detection distance p2, as shown in Figure 1.

A predetermined distance $\rho$ is set between the radiation position 21, where the living body is irradiated with the light, and a detection position 31, where the intensity of the light emitted from the living body (E in Figure 3) is detected, as shown in Figure 3. The thus set predetermined distance $\rho$ suppresses the influence of the light directly emitted from the living body (B in Figure 3), which is the radiated light (A in Figure 3) reflected off the surface of the living body and scatterers in the vicinity of the surface. The radiated light reaches the depth where lipid, such as lipoprotein, is present and is then reflected off the lipid (D in Figure 3) in the blood of the living body.

The light radiator 2 is so fixed that the angle $\theta$ between the optical axis of the light radiator 2 and the surface of the living body E is a predetermined angle greater than or equal to 45° but smaller than or equal to 175°, as shown in Figure 3. The fixed angle of the light radiator 2 can be adjusted by an arbitrary mechanism.

Figure 14 is intended to verify the angle of the light radiator and shows verification of a change in turbidity of the blood and the angle of incidence of the light incident on the living body by using a solid phantom provided with a blood vessel-shaped cavity and injecting blood having a scattering coefficient changed to a variety of values into the cavity. The depth of the cavity is 1 mm. The measured intensity (a.u.) shows the amount of relative change in the optical intensity with respect to the light reception distance.

As a result, a change in turbidity of the blood was measured at the angle of the light radiator ranging from 45° to 175°. Due to the differences in the angle of the light radiator, the measurement range is wide, for example, when the angle of the light radiator is 90°. When the angle of the light radiator is 45°, high resolution is achieved at low values but is saturated in the vicinity of the scatter coefficient of 0.5 /mm. Depending on the characteristics described above, the angle of the light radiator may be selected as appropriate in compatibility with the characteristics of the subject or may be automatically corrected.

The angle $\theta$ of the light radiator in a numerical range of 90° or smaller is preferably greater than or equal to 45° but smaller than or equal to 90°, more preferably, greater than or equal to 45° but smaller than or equal to 65°, and the angle $\theta$ of the light radiator in a numerical range of 90° or greater is preferably greater than or equal to 100° but smaller than or equal to 175°, more preferably, greater than or equal to 115° but smaller than or equal to 175°, still more preferably greater than or equal to 135° but smaller than or equal to 175°. The numerical ranges of 90° or smaller and the numerical ranges of 90° or greater described above may be combined with each other.

After the radiated light is reflectively scattered by the lipid, the optical intensity of the resultant back-scattered light (C in Figure 3) emitted from the living body is detected. Increasing the distance $\rho$ between the radiation position 21 and the detection position 31 increases the optical path length. The number of times of the collision of the light with the lipid therefore increases, so that the detected light is greatly affected by the scattering. Increasing the distance $\rho$ allows the influence of the scattering that is small and has therefore been difficult to detect in related art to be readily captured.

Lipoprotein, which is the target to be measured, has a spherical structure covered with apoprotein and other substances. Lipoprotein is present in the form of a solid-like state in the blood. Lipoprotein has a light reflecting property. In particular, chylomicron (CM), VLDL, and other substances having a large particle diameter and specific gravity contain a large amount of triglyceride (TG) and have a more light-scatterable property. The optical intensity detected by the optical intensity detector 3 is therefore affected by the light scattered by lipoprotein.

In the case where the plurality of detection positions 31 and 32 are set, the detection positions 31 and 32 are not necessarily arranged linearly as long as the positions are arranged at different distances roughly around the radiation position 21, and a circular arrangement, a wavy arrangement, a zigzag arrangement, or any other arrangement can be selected as appropriate. The first radiation detection distance $\rho 1$ from the radiation position 21 to the detection position 31, the second radiation detection distance p2 from the radiation position 21 to the detection position 32, and the gap between the detection positions 331 and 332 are not each limited to a fixed value and may instead be continuously changed.

The configuration of a control system of the blood lipid concentration measurement device 1 will next be described. Figure 2 is a block diagram of the blood lipid concentration measurement device 1 according to the embodiment. A CPU (central processing unit) 41, a ROM (read only memory) 43, a RAM (random access memory) 44, an HDD (hard disk drive) 45, an external I/F (interface) 46, the light radiator 2, and the optical intensity detector 3 are connected to each other via a system bus 42. The CPU 41, the ROM 43, and the RAM 44 form the controller 4.

The ROM 43 stores in advance a program executed by the CPU 41 and a threshold used by the CPU 41.

The RAM 44 has an area where the program executed by the CPU 41 is developed, a variety of memory areas, such as a work area where the program processes data, and other areas.

The HDD 45 stores data in which the correlation between the lipid concentration in the blood of the living body and the turbulence of the optical intensity (amount of turbulence of blood flow) of a plurality of persons is used as a calibration curve.

The external I/F 46 is an interface for communication with an external device, for example, a client terminal (PC). The external I/F 46 only needs to be an interface for data communication with the external device and may be an instrument (such as USB memory) to be locally connected to the external device or a network interface for communication via a network.

The blood lipid concentration measurement device 1 having the configuration described above performs a blood lipid concentration measurement job based on a program set in advance.

The controller 4 calculates a scatter coefficient $\mu s'$ in the living body (including blood, skin, muscle, and other body parts, the same hereinafter) based on the optical intensity detected by the optical intensity detector 3.

The optical intensity detected by the optical intensity detector 3 contains the optical intensity affected by the light scattered by lipoprotein, as described above.

The scatter coefficient $\mu s'$ in the present embodiment is not limited to a quantified efficiency of a typical scatter process and includes a quantified influence of the scattering under a fixed condition in consideration of the scatter phenomenon.

The controller 4 calculates the light intensity ratio or the light intensity difference.

The controller 4 calculates the scatter coefficient $\mu s'$ based on the ratio between the optical intensities detected by a plurality of optical intensity detectors 3. The controller 4 calculates the scatter coefficient $\mu s'$ based on a scattering phenomenon in which the amount of attenuation of the radiated light due to the scattering increases with the distance to a detection position 33.

In the present embodiment, the light radiator 2 radiates continuous light having a predetermined optical intensity, and the scatter coefficient $\mu s'$ is calculated based on the ratio between an optical intensity $R(\rho 1)$ detected by the first optical intensity detector 31 and an optical intensity $R(p2)$ detected by the second optical intensity detector 32 (Expression 1).

$$\text{(Expression 1)}$$

$$\mu s' = R(\rho 1)/R(\rho 2)$$

The controller 4 calculates the scatter coefficient $\mu s'$ based on the difference between the optical intensities detected by the plurality of optical intensity detectors 3. The controller 4 calculates the scatter coefficient $\mu s'$ based on the scattering phenomenon in which the amount of attenuation of the radiated light due to the scattering increases with the distance to the detection position 33.

The controller 4 in the present embodiment calculates the scatter coefficient $\mu s'$ from the difference between the optical intensity $R(\rho 1)$ in the first detection position 331 and the optical intensity $R(p2)$ in the second detection position 332 (Expression 2).

$$\text{(Expression 2)}$$

$$\mu s' = R(\rho 1) - R(\rho 2)$$

The method in accordance with which the controller 4 calculates the scatter coefficient $\mu s'$ is not limited to the calculation methods described above.

The controller 4 calculates the turbulence intensity I (amount of turbulence of blood flow) based on the calculated

scatter coefficient μs'. The method for calculating the turbulence intensity I (amount of turbulence of blood flow) has been described above.

For example, to measure the blood lipid concentration in a specific living body (person A), the result of the measurement of the amount of change in the blood lipid concentration of the person A measured by another blood lipid concentration measurement method, such as blood collection, is compared with the calculated turbulence intensity (amount of turbulence of blood) to create statistical data on the individual A. The lipid concentration can thus be calculated.

Instead, the result of measurement of the blood lipid concentration of the person A measured, for example, by another blood lipid concentration measurement method may be compared with the result of the lipid concentration measurement result obtained by the present apparatus, an error between the lipid concentration obtained by the comparison and the lipid concentration in the statistical data on a typical living body may be calculated, and calibration for correction of the error may be performed to create the statistical data on the individual A.

The concentration and the turbidity used in a clinical site are synonymous with each other, and the concentration in the present embodiment also includes the concept of turbidity. Therefore, the result of the calculation performed by the controller 4 can be not only the concentration but the number of particles per unit quantity, the formazin turbidity, or the amount of change in average lipid particle diameter.

The format of the statistical data is not limited to a specific format, and the statistical data may be classified in terms, for example, of gender, body height, body weight, BMI, or any other factor or may be calculated by using a table, a graph, a function, or any other tool.

The controller 4 can also determine the lipid concentration from a temporal change in the optical intensity (amount of turbulence of blood flow) of the light received by the light intensity detector at a single location.

Phenomena assumed to be an increase in turbulence due to an increase in lipoprotein or a change in the shape of the lipoprotein can be observed in optical measurement based on simple one-point light reception. In the case of one-point light reception, the lipid concentration from a temporal change in the optical intensity (the amount of turbulence of blood flow) is calculated by the expression below.

[Numerical expression 3]

$$X = \frac{1}{T\bar{x}} \int_0^T x \, d\theta$$

T: Measurement time
x: Measured optical intensity
$\bar{x}$: Average of measured optical intensities
θ: Time
X: Turbulence for fixed time = lipid concentration

In the simple one-point light reception, the distance between the light radiator and the light receiver is preferably set at a value approximately ranging from 1.5 to 2 cm. To determine the turbidity by temporal resolution measurement using pulsed light, one-point measurement can also be performed. In this case, the sampling rate employed by the light receiver is more important than the cycle employed by the light source. For example, even in the case of wireless data acquisition, a change in blood turbidity can be measured as long as the sampling rate is at least 250 per second. The time required for the optical intensity detector 3 to measure the optical intensity may be longer than or equal to 1 msec but shorter than or equal to 20 sec.

The blood lipid concentration measurement device 1 according to the present embodiment may include a current applicator that causes pulse current to flow in the living body. Lipid particles are charged, and the zeta potential varies depending on the type of lipoprotein. The current applicator uses the property described above to cause pulsed current to flow from a point outside the body into the body to vibrate CM or VLDL. The scattering coefficient thus changes, whereby the distribution of the lipoprotein can be measured more accurately.

A blood lipid concentration measurement method according to the present embodiment will next be described. Figure 4 is a flowchart of the blood lipid concentration measurement method according to the present embodiment.

In a light radiation step (S101), the light radiator 2 is used to radiate continuous light to the radiation position 21.

In an optical intensity detection step (S102), the first optical intensity detector 31 is used to detect the optical intensity in the first detection position 331, and the second optical intensity detector 32 is used to detect the optical intensity in the second detection position 332. The optical intensities detected in the first detection position 331 and the second detection position 332 are sent to a lipid concentration calculation step.

In the case where a plurality of detection positions 31 are set, the detection positions 31 are not necessarily arranged linearly as long as the positions are arranged at different distances roughly around the radiation position 21, and a circular arrangement, a wavy arrangement, a zigzag arrangement, or any other arrangement can be selected as appropriate. The first radiation detection distance $\rho1$ and the second radiation detection distance p2 from the radiation position 21 to each detection position 31 and the gap between the detection positions 331 and 332 are not each limited to a fixed value and may instead be continuously changed.

In the lipid concentration calculation step (S103), the blood lipid concentration is calculated based on the amount of turbulence of the blood flow. The method for calculating the blood lipid concentration has been described above.

As described above, the blood lipid concentration measurement device and the method therefor according to the present embodiment allow measurement of the blood lipid concentration.

Reference Sings list

    1 Blood lipid concentration measurement device
    2 Light radiator
    3 Optical intensity detector
    4 Controller
    21 Radiation position, 22 Light source
    31 First optical intensity detector, 32 Second optical intensity detector, 33 Detection position
    331 First detection position, 332 Second detection position

**Claims**

1. A blood lipid concentration measurement device comprising:

   a light radiator that radiates light to a living body at a predetermined angle that is an angle between a surface of the living body and an optical axis, the angle being greater than or equal to 45° but smaller than or equal to 175°;
   an optical intensity detector that is located at a predetermined distance from the light radiator and measures an optical intensity of the light reflected off the living body; and
   a controller that calculates an amount of turbulence of blood flow based on the light intensity and calculates a lipid concentration based on the amount of turbulence of blood.

2. The blood lipid concentration measurement device according to claim 1,

   wherein the amount of turbulence of the blood flow is a turbulence intensity, and
   the controller calculates the lipid concentration based on the turbulence intensity.

3. The blood lipid concentration measurement device according to claim 1,

   wherein the amount of turbulence of the blood flow is an amount of change in the optical intensity, and
   the controller calculates the lipid concentration based on the amount of change in the optical intensity.

4. The blood lipid concentration measurement device according to claim 3, wherein time required for the optical intensity detector to measure the amount of change in the optical intensity is longer than or equal to 1 msec but shorter than or equal to 20 sec.

5. The blood lipid concentration measurement device according to any of claims 1 to 4, wherein an angle of the light radiator is adjustable.

6. The blood lipid concentration measurement device according to any of claims 1 to 5, wherein the optical intensity detector is formed of a plurality of optical intensity detectors arranged in positions different from one another.

7. The blood lipid concentration measurement device according to claim 6, wherein the plurality of optical intensity detectors are arranged in a circular or linear arrangement.

8. The blood lipid concentration measurement device according to any of claims 1 to 7, wherein an intensity of the light radiated from the light radiator is greater than or equal to 0.0025 mW but smaller than or equal to 30 mW.

9. A blood lipid concentration measurement method comprising:

a light radiation step of radiating light to a predetermined position on a living body at a predetermined angle that is an angle between a surface of the living body and an optical axis, the angle being greater than or equal to 45° but smaller than or equal to 175°;
an optical intensity detection step of measuring an optical intensity of the light reflected off the living body in a position separate from the predetermined position by a predetermined distance; and
a lipid concentration calculation step of calculating an amount of turbulence of blood flow based on the light intensity and calculating a lipid concentration based on the amount of turbulence of blood.

FIG. 1

FIG. 2

FIG. 3

FIG. 4

FIG. 5

SKIN LAYER

BLOOD LAYER

MUSCLE AND THE LIKE

A = SKIN LAYER
B = SKIN LAYER + BLOOD LAYER
C = SKIN LAYER + BLOOD LAYER + MUSCLE LAYER

FIG. 6

SCATTER COEFFICIENT IN HUNGRY STATE
(FOREARM)

FIG. 7

FIG. 8

AMPLITUDE AT BASE LINE

TIME (5 MINUTES)

FIG. 9

FIG. 10

FIG. 11

FIG. 12

FIG. 13

FIG. 14

## INTERNATIONAL SEARCH REPORT

| | International application No. |
|---|---|
| | PCT/JP2019/045477 |

### A. CLASSIFICATION OF SUBJECT MATTER
Int.Cl. A61B5/1455(2006.01)i, G01N21/17(2006.01)i, G01N21/47(2006.01)i

According to International Patent Classification (IPC) or to both national classification and IPC

### B. FIELDS SEARCHED

Minimum documentation searched (classification system followed by classification symbols)
Int.Cl. A61B5/06-5/22, A61B9/00-10/06, G01N21/00-21/01, G01N21/17-21/61

Documentation searched other than minimum documentation to the extent that such documents are included in the fields searched
Published examined utility model applications of Japan      1922–1996
Published unexamined utility model applications of Japan      1971–2019
Registered utility model specifications of Japan      1996–2019
Published registered utility model applications of Japan      1994–2019

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X<br>A | WO 2018/151022 A1 (MEDICAL PHOTONICS CO., LTD.) 23 August 2018, paragraphs [0001]–[0074], fig. 1–13 & TW 201835553 A, paragraphs [0001]–[0076], fig. 1–13 | 1, 3–9<br>2 |
| X<br>A | WO 2017/199492 A1 (MEDICAL PHOTONICS CO., LTD.) 23 November 2017, paragraphs [0001]–[0179], fig. 1–14 & US 2019/0192056 A1, paragraphs [0001]–[0204], fig. 1–14 & EP 3459457 A1 & CN 109152558 A & KR 10-2019-0008357 A & TW 201740881 A | 1, 3–9<br>2 |

☒ Further documents are listed in the continuation of Box C.    ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 18.12.2019 | 07.01.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

## INTERNATIONAL SEARCH REPORT

| | International application No. |
|---|---|
| | PCT/JP2019/045477 |

C (Continuation). DOCUMENTS CONSIDERED TO BE RELEVANT

| Category* | Citation of document, with indication, where appropriate, of the relevant passages | Relevant to claim No. |
|---|---|---|
| A | WO 2016/208010 A1 (HAMAMATSU PHOTONICS KK) 29 December 2016, paragraphs [0001]-[0058], fig. 1-12 & US 2018/0180539 A1, paragraphs [0001]-[0088], fig. 1-12 & EP 3315943 A1 | 1-9 |
| A | JP 2017-113461 A (TOSHIBA CORPORATION) 29 June 2017, paragraphs [0001]-[0304], fig. 1-23 (Family: none) | 1-9 |
| A | WO 2017/141895 A1 (MEDICAL PHOTONICS CO., LTD.) 24 August 2017, paragraphs [0001]-[0133], fig. 1-19 & US 2019/0046091 A1, paragraphs [0001]-[0170], fig. 1-19 & EP 3417779 A1 & CN 108697388 A & KR 10-2018-0111956 A & TW 201729759 A | 1-9 |

Form PCT/ISA/210 (continuation of second sheet) (January 2015)

**EP 3 884 864 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014087825 A